# EUROPEAN PATENT APPLICATION

(11) **EP 4 652 963 A1**
(43) Date of publication of application: **26.11.2025**
(21) Application number: 24177609.5
(22) Date of filing: 23.05.2024
(51) Int. Cl.: A61C 17/06, G01R 33/34, A61B 5/00, A61B 5/055, G01R 33/20, A61C 1/00

(54) **MEANS FOR REMOVING SALIVA FOR MAGNETIC RESONANCE IMAGING**

(71) Applicant: Siemens Healthineers AG, 91301 Forchheim (DE); Dentsply Sirona Inc., York, PA 17401 (US); Sirona Dental Systems GmbH, 64625 Bensheim (DE); Aarhus Universitet, 8000 Aarhus C (DK)
(72) Inventor: Burzan, Kim, 69488 Birkenau (DE); Greiser, Andreas, 91054 Erlangen (DE); Spin-Neto, Rubens, 8240 Risskov (DK)
(74) Representative: Siemens Healthineers Patent Attorneys

(57) **Abstract**

The invention relates to a saliva suction device (30) for an intraoral radio-frequency coil (41), comprising a body (31) and a suction opening (32) configured to be connected to a vacuum system (50), wherein the body (31) is configured to provide a mechanical connection between the saliva suction device (30) and the intraoral radio-frequency coil (41), wherein the intraoral radio-frequency coil (41) is configured to receive magnetic resonance signals from a dental region of a patient (15), and wherein the suction opening (32) is configured to remove saliva from an oral cavity of the patient (15) during a magnetic resonance measurement. The invention also relates to a combined intraoral suction device (40) comprising an inventive saliva suction device (30) and a magnetic resonance device (10) comprising a saliva suction device (30).

## Description

*Independent of the grammatical term usage, individuals with male, female or other gender identities are included within the term.*

The invention relates to means for removing saliva from an oral cavity of a patient during a magnetic resonance measurement.

In recent times, magnetic resonance imaging is gaining increasing interest for applications in dental imaging (e. g. imaging of teeth and a surrounding dentomaxillofacial anatomy), as it enables radiation-free imaging and provides a superb soft tissue contrast, allowing for a differential diagnosis of tissue status and visualization of inflammation or irreversible tissue alterations.

For a magnetic resonance measurement of a dental region, it is recommended to use dedicated receive coils which can be closely arranged to the target anatomy, e. g. the dentition or the jaw region of a patient, to improve the diagnostic image quality. The receive coils can be designed as extraoral coils (EOC), configured to be placed in vicinity to an external facial surface of a patient, or as intraoral coils (IOC), configured to be arranged inside the patient's mouth or oral cavity. While intraoral coils can typically be arranged closer to the teeth, the patient may react to intraoral coils with nausea, retching, and/or an increased urge to swallow. Since saliva is naturally accumulating in the oral cavity, an increasing urge to swallow may also arise as a side effect of magnetic resonance measurements during which the patient is not allowed to move or swallow. A swallowing motion may have an adverse effect on magnetic resonance images, as the acquisition of image data typically takes several minutes and any motion within the imaging volume causes image artifacts, such as ghosting or blurring. Furthermore, swallowing motions can lead to suboptimal coverage of relevant structures in the diagnostic sequences, for example if the patient swallows between the acquisition of scout images and subsequent pulse sequences.

It is an object of the invention to facilitate a workflow associated with an acquisition of magnetic resonance signals of a dental region of a patient and improve a quality of images based on the magnetic resonance data received from the dental region of the patient.

This object is achieved by a saliva suction device, a combined intraoral suction device, and a magnetic resonance device according to the invention. Further advantageous embodiments are specified in the dependent claims.

The inventive saliva suction device for an intraoral radio-frequency coil comprises a body and a suction opening configured to be connected to a vacuum system.

Preferably, the saliva suction device is configured for use with an intraoral radio-frequency coil. Particularly, the saliva suction device may be configured to mechanically connect to an intraoral radio-frequency coil during a magnetic resonance measurement. According to the invention, the saliva suction device is configured to remove saliva from an oral cavity of the patient during a magnetic resonance measurement.

The body of the saliva suction device may define an outer shape of the saliva suction device. Preferably, the body is shaped in such a way to allow for an insertion of at least a part of the saliva suction device into the oral cavity or mouth of the patient. Particularly, the body may be shaped in such a way to allow for a part of the saliva suction device comprising one or more suction openings to be inserted into the oral cavity of the patient. It is conceivable that the body is shaped in such a way that the one or more suction openings are arranged between a tooth and an inner surface of a cheek and/or between a tooth and a tongue, when the saliva suction device is properly arranged inside the oral cavity of the patient.

The suction opening may be arranged on a surface of the body of the saliva suction device. Preferably, the saliva suction device comprises a plurality of suction openings configured to fluidly connect to a vacuum system. For example, one or more suction openings of the saliva suction device may be configured to fluidly connect to a fluid chamber and/or a fluid pipe fluidly connected to the vacuum system. It is conceivable that each suction opening forms a hole or channel in a wall of the body of the saliva suction device. The holes or channels may be connected to one or more fluid chambers within the body of the saliva suction device and/or a fluid pipe fluidly connected to the vacuum system.

The vacuum system may comprise a conveying unit, such as a pump or compressor, configured to provide a vacuum or negative pressure within a fluid pipe fluidly connected to the one or more suction openings. According to an embodiment, the conveyor unit is fluidly connected to the one or more suction openings via one or more fluid pipes, hoses, or fluid chambers. The saliva suction device may be configured to transport saliva and/or other fluids located at the one or more suction openings towards the vacuum system. Particularly, the saliva suction device may be configured to remove saliva and/or other fluids from the oral cavity of the patient.

According to the invention, the body of the saliva suction device is configured to provide a mechanical connection between the saliva suction device and the intraoral radio-frequency coil,

For example, a part of the body of the saliva suction device may be shaped in such a way to mechanically engage with a part of the intraoral radio-frequency coil and provide the mechanical connection between the saliva suction device and the intraoral radio-frequency coil. It is conceivable that the body of the saliva suction device is configured to attach to the intraoral radio-frequency coil.

At least a section of the body of the saliva suction device may correspond to a shape of the intraoral radio-frequency coil. For example, at least a section of the body may be shaped in such a way to conform to a shape of a section of the intraoral radio-frequency coil. Preferably, the body of the saliva suction device is shaped to accommodate at least a section of the intraoral radio-frequency coil. It is also conceivable that the body comprises a retaining element configured to hold or maintain the intraoral radio-frequency coil in a predefined relative position to the saliva suction device. According to an embodiment, the body of the saliva suction device is configured to attach to the intraoral radio-frequency coil in such a way to prevent undeliberate movement of the intraoral radio-frequency coil relative to the saliva suction device.

The intraoral radio-frequency coil is configured to receive magnetic resonance signals from a dental region of a patient.

The intraoral radio-frequency coil may comprise one or more antennas configured to receive magnetic resonance signals. An antenna may represent a coupling element between electromagnetic waves guided in a signal conductor and unguided electromagnetic waves, i. e. electromagnetic waves in a free space. The one or more antennas of the intraoral radio-frequency coil are preferably configured to receive electromagnetic waves in a frequency range of a magnetic resonance measurement, particularly a magnetic resonance frequency of a magnetic resonance active atomic nucleus. For example, an electromagnetic wave with a frequency between 1 and 500 MHz, preferably between 10 and 300 MHz, may be considered as a magnetic resonance signal. The magnetic resonance signal of common atomic nuclei can have a low power of a few microwatts to several milliwatts.

The intraoral radio-frequency coil may comprise at least one transmit antenna configured to emit a high-frequency signal into the dental region of the patient. The high-frequency signal emitted by the at least one transmit antenna can, for example, be in a power range between several watts and several kilowatts, depending on the basic magnetic field of a magnetic resonance device.

The one or more antennas of the intraoral radio-frequency coil may comprise or consist of a signal conductor. A signal conductor may comprise or consist of an electrically conductive wire. The electrically conductive wire of the signal conductor may have an oval or polygonal cross-section suitable for permanently transmitting a power specified above. It is also conceivable that the signal conductor is configured as a conducting path on a printed circuit board. Such a signal conductor may have an approximately rectangular cross section. The signal conductor can be made of copper. However, the signal conductor may also comprise or consist of other electrically conductive metals, such as aluminium, platinum, gold, or the like.

Preferably, the one or more antennas of the intraoral radio-frequency coil comprise a protective element configured to protect the patient from electric shocks and/or burns. For example, the protective element may be embodied as a coating and/or a cladding covering a signal conductor and/or an antenna of the intraoral radio-frequency coil. The protective element may comprise or consist of any suitable plastic, such as polytetrafluoroethylene (PTFE) or various polysiloxanes.

During a magnetic resonance measurement, a current can be induced in a signal conductor of the intraoral radio-frequency coil by nuclear magnetic resonance. The current can be detected as a magnetic resonance signal via a dedicated receiving unit or radio-frequency control unit of a magnetic resonance device. In contrast, the magnetic resonance device may be configured to supply a signal conductor of a transmit antenna of the intraoral radio-frequency coil with an alternating current to emit a high-frequency signal, the so-called B1 magnetic field, into the dental region of the patient.

It is conceivable that the one or more antennas of the intraoral radio-frequency coil are electrically connected to a radio-frequency control unit of a magnetic resonance device via an electrical signal connection. Such an electrical signal connection may comprise an electrical cable, particularly a coaxial cable, having a shield to avoid electromagnetic interference from the environment.

The intraoral radio-frequency coil may comprise an electronic circuit electrically connected to the one or more antennas. The electronic circuit may comprise an electronic component or a combination of electronic components, such as a transistor, a resistor, a capacitor, a diode, a conductor track, and the like. Particularly, the electronic circuit may comprise a protective circuit configured to protect the one or more antennas against electrical overload. Furthermore, the electronic circuit may contain a high share of non-magnetic materials as well as corresponding standing wave barriers and/or baluns to avoid undesirable effects, such as magnetic attraction forces, standing waves, heating, or the like. In a preferred embodiment, the electronic circuit comprises a printed circuit board (PCB) or a comparable substrate, which is suitable for receiving the electronic components in a predetermined position relative to one another.

The intraoral radio-frequency coil may comprise a substantially flat or plane body configured to be arranged in an occlusal plane between the upper dental arch and the lower dental arch of the patient. However, it is also conceivable that the intraoral radio-frequency coil is shaped in such a way to encompass a section of a dental arch from at least two different sides. In a preferred embodiment, a shape of the intraoral radio-frequency coil or the one or more antennas of the intraoral radio-frequency coil corresponds to or matches a shape of a dental arch of the patient.

According to the invention, the suction opening is configured to remove saliva from an oral cavity of the patient during a magnetic resonance measurement.

Preferably, the suction opening is configured to remove saliva from an oral cavity of the patient during a magnetic resonance measurement when the saliva suction device is attached to the intraoral radio-frequency coil and the intraoral radio-frequency coil is properly arranged within the oral cavity of the patient.

As described above, the body of the saliva suction device may be shaped in such a way to allow insertion of at least a part of the saliva suction device comprising one or more suction openings into the oral cavity of the patient. The one or more suction openings may be fluidly connected to the vacuum system via channels and/or fluid pipes. Thus, the one or more openings may be configured to remove saliva and/or other fluids in vicinity to the one or more suction openings from the oral cavity of the patient.

The inventive saliva suction device may comprise a plurality of suction openings distributed regularly or irregularly over a surface of the body of the saliva suction device. The body of the saliva suction device may form a fluid chamber connecting the one or more suction openings to the vacuum system.

The inventive saliva suction device may represent an attachment for an intraoral radio-frequency coil. However, the saliva suction device may also be configured to be used as a standalone device. For example, the saliva suction device may be configured to remove saliva from the oral cavity of the patient without being attached to an intraoral radio-frequency coil. In a preferred embodiment, the body of the saliva suction device is structurally stable or self-supporting. Thus, the inventive saliva suction device may favourably allow for a removal of saliva from the oral cavity of the patient, even if no intraoral radio-frequency coil is used.

A saliva suction device according to the invention may favourably allow for a removal of saliva during a magnetic resonance measurement. Thus, an urge to swallow of the patient during the magnetic resonance measurement may favourably be reduced or averted.

In providing a saliva suction device fitted or attached to an intraoral radio-frequency coil, an insertion of multiple separate devices into a patient's mouth may favourably be avoided. Thus, a comfort of the patient may be increased and a risk of termination of the magnetic resonance measurement by the patient can favourably be reduced.

Furthermore, an inventive saliva suction device attached to an intraoral radio-frequency coil may favourably prevent a relative movement between the saliva suction device and the intraoral radio-frequency coil. Thus, a process of properly positioning the intraoral radio-frequency coil, but also the saliva suction device, for the magnetic resonance measurement may favourably be facilitated. Particularly, a mechanical connection between the saliva suction device and the intraoral radio-frequency coil may ensure a correct position of the intraoral radio-frequency coil and the one or more suction openings of the saliva suction device relative to a dental arch of the patient.

According to an embodiment of the inventive saliva suction device, the body is configured to encompass at least a part of the intraoral radio-frequency coil and/or provide a protective cover for at least a part of the intraoral radio-frequency coil.

It is conceivable that the body of the saliva suction device is configured to cover or encase at least a section of the intraoral radio-frequency coil, which is to be arranged within the oral cavity of the patient. The body of the saliva suction device may comprise or consist of an elastic material and/or a soft material. A body made from an elastic and/or soft material may be configured to be stretched over at least part of the intraoral radio-frequency coil and/or take a shape of at least part of the intraoral radio-frequency coil. In providing a saliva suction device comprising a body made from a soft or elastic material, damage to sensitive tissues within the oral cavity of the patient via the saliva suction device and/or the intraoral radio-frequency coil may favourably be reduced or avoided.

Preferably, at least a section of the body configured to be arranged within the oral cavity of the patient comprises rounded edges to avoid injury of the patient. It is also conceivable that at least a section of the body configured to be arranged within the oral cavity of the patient comprises or consists of an electrical insulator. An electrical insulator may favourably provide a protection against electrical shocks or burns caused by a direct contact between tissue and an antenna of the intraoral radio-frequency coil.

The body of the saliva suction device may be configured to prevent the intraoral radio-frequency coil from making a direct contact with the teeth of the patient. For example, at least a section of the body of the saliva suction device may be arranged between a tooth and the intraoral radio-frequency coil, when the saliva suction device and the intraoral radio-frequency coil are properly arranged within the oral cavity of the patient for a magnetic resonance measurement of the dental region. Thus, damage to the intraoral radio-frequency coil by the teeth of the patient may favourably be prevented and a longevity of the intraoral radio-frequency coil may favourably be improved. Moreover, a provision of additional protective elements between the intraoral radio-frequency coil and a tissue within the oral cavity of the patient may favourably be avoided.

A saliva suction device covering or encasing at least a section of the intraoral radio-frequency coil may favourably act as a barrier against dirt, germs and/or body fluids. Thus, cleaning or sterilization of the intraoral radio-frequency coil after use may be facilitated or even omitted.

According to a further embodiment of the inventive saliva suction device, the body is shaped in such a way to encompass at least a part of the intraoral radio-frequency coil and to provide a form-locking and/or force locking mechanical connection with the part of the intraoral radio-frequency coil.

For example, the body of the saliva suction device may be configured to enclose or encompass at least a section of the intraoral radio-frequency coil, thus providing a form-locking mechanical connection between the saliva suction device and the intraoral radio-frequency coil. However, the body may also comprise a retaining element, a protruding section and/or or a section configured to surround, clamp or otherwise mechanically engage with a section of the intraoral radio-frequency coil to provide a form-locking and/or a force-locking mechanical connection between the saliva suction device and the intraoral radio-frequency coil.

According to an embodiment, a section of the body of the saliva suction device configured to accommodate and/or encompass a section of the intraoral radio-frequency coil is slightly undersized with respect to the section of the intraoral radio-frequency coil accommodated and/or encompassed by the section of the body of the saliva suction device. Thus, the section of the body of the saliva suction device configured to accommodate and/or encompass the section of the intraoral radio-frequency coil may be configured to provide a press connection or a clamp connection with the section of the intraoral radio-frequency coil accommodated and/or encompassed by the body of the saliva suction device.

Preferably, the body of the saliva suction device comprises or consists of a flexible or elastic material configured to accommodate at least a part of the intraoral radio-frequency coil by stretching and holding it in place via elastic restoring forces. Examples of suitable flexible or elastic materials are natural or synthetic elastomers or other elastic polymers or plastic materials which can be deformed with manual effort.

A saliva suction device configured to provide a form-locking and/or force locking mechanical connection with the intraoral radio-frequency coil may favourably facilitate a process of attaching the saliva suction device to the intraoral radio-frequency coil. Particularly, an inventive saliva suction device may favourably be attached to the intraoral radio-frequency coil without need for dedicated tools or other aids.

Furthermore, in providing a saliva suction device configured to accommodate and/or conform to an intraoral radio-frequency coil, a footprint or dimension of components required to be inserted into the patient's mouth may favourably be reduced.

According to a preferred embodiment of the inventive saliva suction device, the body comprises a receiving section shaped complementary to a section of the intraoral radio-frequency coil in such a way to provide a form-locking mechanical connection between the section of the intraoral radio-frequency coil and the receiving section of the saliva suction device.

Preferably, the body of the saliva suction device comprises or consists of a flexible or elastic material according to an embodiment described above. Particularly, a dimension of the receiving section may be slightly undersized in comparison to a dimension of the section of the intraoral radio-frequency coil to be accommodated within the receiving section of the saliva suction device.

For example, the receiving section may comprise a rail or a guide element allowing the section of the intraoral radio-frequency coil to slide into the receiving section of the saliva suction device. However, the receiving section may also be configured as a cradle, particularly an indentation, a depression, or a recess, in a surface of the body of the saliva suction device. Preferably, such a cradle in the surface of the body of the saliva suction device extends to an edge of the body of the saliva suction device. Thus, the section of the intraoral radio-frequency coil may be slid into the receiving section of the saliva suction device from a side. Furthermore, electrical cables of the intraoral radio-frequency coil may be routed out of the body of the saliva suction device without requiring an additional cable feed-through. However, the receiving section may also be configured in such a way to allow for the section of the intraoral radio-frequency coil to be pushed into the receiving section from any direction.

According to an embodiment, the receiving section is shaped complementary to a section of the intraoral radio-frequency coil in such a way to allow for a form-locking mechanical connection between the section of the intraoral radio-frequency coil and the receiving section of the saliva suction device via a sliding and/or pushing motion.

The receiving section may be configured to accommodate a main surface of the intraoral radio-frequency coil. However, the receiving section may also be configured to accommodate a side and/or an edge of the intraoral radio-frequency coil. Preferably, the receiving section is shaped in accordance with a section of the intraoral radio-frequency coil to be attached to the saliva suction device. For example, the receiving section may be shaped in such a way to conform to an outer surface of the section of the intraoral radio-frequency coil to be attached to the saliva suction device.

In providing a saliva suction device comprising a receiving section, a process of attaching the saliva suction device to an intraoral radio-frequency coil may be facilitated. Furthermore, a correct relative orientation between the saliva suction device and the intraoral radio-frequency coil may favourably be recognized visually by a user based on the shape of the receiving section.

In a further embodiment, the inventive saliva suction device comprises a retaining element configured to hold the intraoral radio-frequency coil in a predefined relative position to the saliva suction device.

The retaining element may be configured as a clipping element, a clamping element, a locking element, a sliding element, a hook-and-loop fastener, or the like. For example, the retaining element may comprise a pin, a bolt, or a cover configured to secure the intraoral radio-frequency coil to the saliva suction device.

According to an embodiment, the retaining element comprises a hinge, a movable joint and/or a flap configured to arrange the retaining element in an "open" position and a "closed" position. For example, the retaining element may provide access to the receiving section of the saliva suction device, particularly a section of the intraoral radio-frequency coil arranged within the receiving section of the saliva suction device, when arranged in the "open" position. When arranged in the "closed" position, the retaining element may secure the intraoral radio-frequency coil in a predefined relative position to the saliva suction device.

A saliva suction device comprising a retaining element may provide an improved mechanical connection between the saliva suction device and an intraoral radio-frequency coil. Thus, any undesired detachment of the intraoral radio-frequency coil from the saliva suction device in the oral cavity of the patient may favourably be avoided, while still allowing for an easy and/or time-efficient disassembly after the magnetic resonance measurement has been carried out and the intraoral radio-frequency coil has been withdrawn from the oral cavity of the patient.

According to an embodiment of the inventive saliva suction device, the body comprises or consists of an elastic material configured to allow the body to reversibly deform under manual effort.

The body of the saliva suction device may comprise or consist of an elastic material according to an embodiment described above. For example, the body may consist of a natural or synthetic elastomer.

The body is configured to attach to and/or detach from the intraoral radio-frequency coil under reversible deformation. Preferably, the body or the receiving section of the saliva suction device is slightly undersized in comparison to a section of the intraoral radio-frequency coil to be attached to the saliva suction device. It is conceivable that the elastic material of the body is configured to be stretched over a part or section of the intraoral radio-frequency coil, thus holding the intraoral radio-frequency coil in place via elastic restoring forces of the elastic material.

Furthermore, at least a section of the body comprising or consisting of an elastic material may be configured to act as a hinge or joint for a retaining element. For example, a section of the body comprising the elastic material may be configured to be deformed or bent in such a way to variably arrange the retaining element in an "open" position or a "closed" position in accordance with an embodiment described above.

It is also conceivable that a section of the body comprising or consisting of an elastic material is configured to wrap around or encompass an edge or section of the intraoral radio-frequency coil, thus providing a form-locking and/or force-locking connection between the saliva suction device and the intraoral radio-frequency coil.

A saliva suction device comprising a reversibly deformable body may favourably facilitate a process of attaching the saliva suction device to an intraoral radio-frequency coil.

According to an embodiment, the intraoral radio-frequency coil may comprise a body configured to mechanically engage with a part of the saliva suction device. Particularly, a part of the body of the saliva suction device may be shaped complementary to a part of the intraoral radio-frequency coil. It is also conceivable that a part of the intraoral radio-frequency coil is shaped and/or configured in such a way to mechanically engage with a part of the body of the saliva suction device to provide the mechanical connection between the saliva suction device and the intraoral radio-frequency coil.

According to an embodiment, the inventive saliva suction device comprises a portable vacuum system fluidly connected to the suction opening.

The portable vacuum system may be fluidly connected to one or more suction openings of the saliva suction device in accordance with an embodiment described above.

A portable vacuum system may be configured to be lifted and/or carried by a user, such as a member of a medical staff, a technician, or the patient. Particularly, the portable vacuum system may be configured to be arranged in different places, e. g. different locations in an examination room.

In a preferred embodiment, the portable vacuum system is configured to be detachable from the saliva suction device and/or a fluid pipe connected to the saliva suction device. For example, the portable vacuum system may comprise a quick connector or a detachable pipe connection configured to provide a fluid connection with the one or more suction openings of the saliva suction device.

It is conceivable that the fluid pipes or fluid connections connecting the portable vacuum system to the one or more suction openings of the saliva suction device are at least partially embedded within a patient table, or a patient positioning device of a magnet resonance device comprising the intraoral radio-frequency coil. Thus, an irritation of the patient by fluid pipes or fluid connections within an imaging region of a magnetic resonance device may favourably be avoided, while still allowing for a quick and/or easy set-up of the saliva suction device for a magnetic resonance measurement of the dental region of the patient.

A portable vacuum system may favourably be removed from a patient table or a patient positioning device when other body parts of the patient, e. g. body parts which are insensitive to a swallowing motion, are examined. Thus, an imaging region and/or patient access region of a magnetic resonance device may be kept as free as possible, which facilitates an interaction with the patient.

The inventive combined intraoral suction device comprises a saliva suction device and an intraoral radio-frequency coil attachable to the saliva suction device. The intraoral radio-frequency coil is configured to receive magnetic resonance signals from a dental region of a patient when the combined intraoral suction device is properly arranged within an oral cavity of the patient for a magnetic resonance measurement of the dental region.

The saliva suction device and the intraoral radio-frequency coil may be configured in accordance with an embodiment described above. Particularly, the saliva suction device may be reversibly attached to the intraoral radio-frequency coil via a form-locking and/or a force-locking mechanical connection.

The inventive combined intraoral suction device shares the advantages of the inventive saliva suction device according to an embodiment described above.

According to an embodiment of the inventive combined intraoral suction device, the body of the saliva suction device and the intraoral radio-frequency coil are permanently or irreversibly attached to one another. For example, the body of the saliva suction device and the intraoral radio-frequency coil may form a coherent or cohesive structure. Preferably, the saliva suction device comprises a body with one or more suction openings configured in accordance with an embodiment described above. The combined intraoral suction device may be shaped in such a way to allow for a section of the saliva suction device comprising the one or more suction openings to be arranged between a tooth and an inner surface of a cheek and/or a tooth and a tongue of the patient, when the combined intraoral suction device is properly arranged within the oral cavity of the patient.

The combined intraoral suction device, the saliva suction device, but also the intraoral radio-frequency coil, may be considered properly arranged inside the oral cavity of the patient, when the intraoral radio-frequency coil attached to the saliva suction device is in contact or properly aligned with at least one dental arch of the patient. For example, a signal conductor or antenna of the intraoral radio-frequency coil may be arranged in an occlusion plane between the two dental arches of the patient. Particularly, the signal conductor or antenna may be arranged in such a way to match and/or cover a section of a dental arch or an entire dental arch of the patient. Preferably, the signal conductor or antenna is arranged in such a way to enable an acquisition of magnetic resonance signals from a specific section of the dental arch or the entire dental arch of the patient.

In providing a combined intraoral suction device comprising an intraoral radio-frequency coil permanently attached to a saliva suction device, a process of attaching and/or detaching of the intraoral radio-frequency coil from the saliva suction device may favourably be omitted. Thus, a time required for preparing a patient for a magnetic resonance measurement of the dental region may favourably be reduced.

The inventive magnetic resonance device is configured to perform a magnetic resonance measurement of a dental region of a patient positioned within an imaging region of the magnetic resonance device.

The magnetic resonance device may be configured to acquire magnetic resonance image data, particularly diagnostic magnetic resonance image data, from the patient positioned within the imaging region. The patient may be a human or an animal. In a preferred embodiment, the magnetic resonance device is configured to perform a magnetic resonance measurement of a dental region of the patient. Particularly, the magnetic resonance device may be configured to acquire magnetic resonance signals from the dental region of the patient via the intraoral radio-frequency coil.

The inventive magnetic resonance device may represent a closed bore scanner. A closed bore scanner may comprise a substantially cylindrical bore circumferentially enclosing the imaging region. Particularly, the closed bore scanner may comprise one or more solenoidal superconducting magnet coils circumferentially encompassing the imaging region along an axial direction or an axis of rotational symmetry of the cylindrical bore. The one or more superconducting magnet coils may comprise a superconducting wire having a negligible electrical resistance at (or below) a superconducting temperature. A direction of a main magnetic field provided via the one or more superconducting magnet coils may be oriented substantially in parallel to a direction of access of the patient to the imaging region and/or the axial direction of the cylindrical bore.

The inventive magnetic resonance device may comprise further components required for a proper operation of the magnetic resonance device. For example, the magnetic resonance device may comprise an outer vacuum chamber, a magnet support structure, a thermal shield, a cryocooler, and the like. In certain embodiments, the magnetic resonance device comprises a cryogen vessel and/or a thermal buffer configured for storing or preserving a fluid, particularly a cryogen, at a predefined temperature level. Preferably, the fluid or cryogen exhibits a low boiling point. Examples of suitable fluids or cryogens are Argon, Nitrogen, Neon, Helium, or the like. The predefined temperature level may substantially correspond to a superconducting temperature of a superconducting magnet coil.

A cryocooler may be configured to cool the one or more superconducting magnet coils of the magnetic resonance device and/or maintain the one or more superconducting magnet coils at a temperature level close to the superconducting temperature. The superconducting coils, the thermal shield, the magnet support structure, and/or the cryogen vessel may be thermally connected to the cryocooler via a solid thermal conductor, a convection loop and/or a heat pipe.

According to the invention, the magnetic resonance device comprises a control unit, a saliva suction device, and a vacuum system connected to a suction opening of the saliva suction device. The suction opening is configured to remove saliva from the oral cavity of the patient.

Preferably, the saliva suction device is configured to remove saliva from a patient's mouth during a magnetic resonance measurement of a dental region of the patient. The saliva suction device and the vacuum system may be configured in accordance with an embodiment described above.

The control unit is configured to activate and deactivate the vacuum system. The control unit may form a part of a main control unit of the magnetic resonance device. However, the control unit may also form an independent or standalone component connected to the main control unit via a suitable signal connection.

The control unit may be connected to the vacuum system via a suitable signal connection. Particularly, the control unit may be configured to output a control signal to activate and deactivate the vacuum system. Thus, the control unit may control a pressure at the one or more suction openings of the saliva suction device. For example, the control unit may be configured to switch-off or pause the vacuum system and switch-on the vacuum system via dedicated control signals. Particularly, the control unit may be configured to activate and deactivate the vacuum system during the magnetic resonance measurement.

A magnetic resonance device comprising a saliva suction device may allow for a removal of saliva from an oral cavity of a patient during a magnetic resonance measurement. Thus, movement and/or imaging artifacts related to swallowing of saliva may favourably be reduced or avoided.

According to an embodiment of the inventive magnetic resonance device, the control unit is configured to activate and deactivate the vacuum system in dependence of an imaging sequence of the magnetic resonance measurement.

The control unit may be configured to output control signals for activating and deactivating the vacuum system in dependence of an imaging sequence of the magnetic resonance device. For example, the control unit may be configured to switch-off or pause the vacuum system before or during an acquisition of magnetic resonance signals. However, the control unit may also be configured to switch-on or activate the vacuum system during a period in which no magnetic resonance signals are acquired, between a first acquisition of magnetic resonance signals and a second acquisition of magnetic resonance signals, and/or after an acquisition of magnetic resonance signals has finished.

An inventive magnetic resonance device may favourably allow for a removal of saliva to be coordinated or timed in dependence of an acquisition of magnetic resonance signals of an imaging sequence of a magnetic resonance measurement. Thus, a motion associated with a removal of saliva or fluid from the oral cavity of the patient may be avoided, particularly shortly before and/or during an acquisition of magnetic resonance signals, but also repetitively over the course of the magnetic resonance measurement. As a result, an occurrence of motion artifacts may be reduced and/or a quality of magnetic resonance images based on the acquired magnetic resonance signals may be improved.

According to an embodiment of the inventive magnetic resonance device, the saliva suction device forms a part of a combined intraoral suction device according to an embodiment described above.

The saliva suction device may be configured to reversibly attach to an intraoral radio-frequency coil of the magnetic resonance device in accordance with an embodiment described above. However, the saliva suction device may also be permanently or irreversibly attached to an intraoral radio-frequency coil of the magnetic resonance device according to an embodiment described above.

The inventive magnetic resonance device shares the advantages of the inventive combined intraoral suction device.

According to an embodiment of the inventive magnetic resonance device, the control unit is configured to automatically deactivate the vacuum system for a duration of an acquisition of magnetic resonance signals.

Preferably, the control unit is also configured to reactivate the vacuum system after the magnetic resonance signals have been acquired. The control unit may be configured to control the vacuum system by outputting a control signal to the vacuum system via a suitable signal connection (e. g. a wired or wireless connection). In deactivating the vacuum system, the negative pressure at the one or more suction openings may cease partially or completely (i. e. the absolute pressure at the one or more suction openings may rise or reach a level of atmospheric pressure). Preferably, the negative pressure at the one or more suction openings is reinstated by reactivating the vacuum system via the control system after the magnetic resonance signals have been acquired.

The control unit may be configured to deactivate the vacuum system for several hundred milliseconds, a fraction of a second, several seconds, a fraction of a minute, or several minutes.

In providing a control unit configured to automatically deactivate the vacuum system for a duration of an acquisition of magnetic resonance signals, image artifacts caused by a flow of saliva and/or air in vicinity to the one or more suction openings may favourably be reduced or avoided.

According to a further embodiment of the inventive magnetic resonance device, the control unit is configured to activate or deactivate the vacuum system in dependence of a trigger signal provided with the imaging sequence, wherein the trigger signal defines one or more time periods during the imaging sequence in which the vacuum system is active or inactive.

The trigger signal may determine or designate a point in time at which the vacuum system is to be switched-on or switched-off. The trigger signal may trigger the control unit to output a control signal to the vacuum system in accordance with an embodiment described above.

The control unit and/or a processing unit connected to or forming a part of the control unit may be configured to determine a trigger signal for an imaging sequence to be carried out. Particularly, the control unit and/or processing unit may be configured to determine a trigger signal for an imaging sequence configured for acquiring magnetic resonance signals of the dental region of the patient. However, it is also conceivable that the trigger signal forms a part of the imaging sequence. Furthermore, the trigger signal may be determined by a predetermined time-schedule of an imaging sequence. Preferably, the control unit comprises a logic unit, particularly a transistor-transistor logic (TTL), configured to provide or output the trigger signal of the imaging sequence. It is conceivable that one or more trigger signals are provided with an imaging sequence.

Preferably, the imaging sequence is configured to acquire magnetic resonance signals of a dental region, particularly a dental arch, a tooth, several teeth, a jaw, and/or a mandible, of the patient. The imaging sequence may have a very short echo times to compensate for a short T2 relaxation time of spins of hard tissue, such as dentin or enamel of a tooth. Very short echo times can be less than 150 µs or less than 70 ps. Particularly, the imaging sequence may comprise a FLASH (fast low-angle shot) or UTE (ultra-short echo time) sequence. However, it is also conceivable that the imaging sequence comprises a longer echo time, such as a TSE (turbo spin echo) sequence. Imaging sequences with short echo times may allow for a detection of magnetic resonance signal of hard tissue, whereas imaging sequences with a longer echo time may result in a lack of signal from hard tissue. In image data acquired via imaging sequences with longer echo times, the teeth can be differentiated, for example, by a lack of signal intensity in contrast to a surrounding tissue.

During an imaging sequence, the intraoral radio-frequency coil may be positioned directly adjacent to a tooth and/or a dental arch of the patient to capture magnetic resonance signals from one or more teeth of the patient. Preferably, the intraoral radio-frequency coil is arranged in the occlusal plane between the dental arches of the patient.

In providing a control unit configured to activate or deactivate the vacuum system in dependence of a trigger signal provided with an imaging sequence, an activation or deactivation of the vacuum system may favourably be coordinated with a predetermined schedule of a specific imaging sequence. Thus, a duration of the magnetic resonance measurement may favourably be reduced.

Furthermore, an inventive magnetic resonance device may be configured to take into account a dynamic of the vacuum system, such as a time required for raising or lowering the negative pressure at the one or more suction openings to a desired level. Thus, an occurrence of image artifacts may favourably be reduced or avoided and/or an efficiency of removal of saliva from the oral cavity of the patient may be improved.

In a preferred embodiment of the inventive magnetic resonance device, the control unit is configured to pause an acquisition of magnetic resonance signals after a predetermined time interval has passed, particularly a time interval of less than 30 s, less than 60 s, less than 90 s, less than 120 s, less than 150 s, less than 180 s, less than 210 s, less than 240 s, less than 270 s, or less than 300 s, and activate the vacuum system for a predetermined period of time before resuming the acquisition of magnetic resonance signals.

The control unit may be configured to pause the magnetic resonance measurement, particularly an imaging sequence and/or an acquisition of magnetic resonance signals, after the predetermined time interval has passed. The predetermined time interval may be defined by a period of time after which a removal of saliva must be initiated or repeated. Particularly, the predetermined time interval may be characterized by an amount of time after which a limit amount of saliva is typically released within the oral cavity of the patient which invokes an urge to swallow.

The control unit may be configured to interrupt or suspend an execution of the magnetic resonance measurement and/or the imaging sequence. It is conceivable that a processing unit of the control unit is configured to calculate breaks or pauses within an imaging sequence, which ensure compliance with the predetermined time interval without requiring a direct interruption of an acquisition of magnetic resonance signals. For example, the imaging sequence may be interrupted in between different acquisitions of magnetic resonance signals, during or after an emission of gradient pulses, during a relaxation time, but also in between breaks required for ensuring or monitoring a specific absorption rate (SAR) limit and/or adjusting imaging parameters (e. g. parameters of a scan or imaging protocol or a parameter related to a position and/or orientation of the intraoral radio-frequency coil), or the like. It is also conceivable that the control unit is configured to extend or prolong a break between two subsequent imaging sequences or scans for the predetermined time interval.

In a preferred embodiment, the control unit is configured to interrupt or pause the magnetic resonance measurement in between adjustment scans and/or imaging scans and activate the vacuum system for a predetermined period of time. An adjustment scan may comprise or represent a navigator scan, a projection scan, or any other coarse-resolution and/or time-efficient scan configured to determine a spatial position of one or more features of the dental regions (e. g. landmarks or anatomic markers) and/or a relative spatial arrangement of the one or more features. Particularly, an adjustment scan may be used for planning of a field of view of a subsequent imaging scan. An imaging scan may be configured to acquire a magnetic resonance image, particularly a high-resolution magnetic resonance image, from at least a part of the dental region of the patient. The magnetic resonance measurement may comprise one or more adjustment scans, but also one or more imaging scans. In conducting a plurality of imaging scans, a plurality of magnetic resonance images (also referred to as "averages") may be acquired, which may be used to enhance a quality or accuracy of the acquired magnetic resonance data of the dental region. The control unit may be configured to interrupt the magnetic resonance measurement in between two adjustment scans, in between an adjustment scan and an imaging scan, but also in between two or more imaging scans, and activate the vacuum system for a predetermined period of time before resuming the magnetic resonance measurement. Particularly, the control unit may be configured to interrupt the magnetic resonance measurement and activate the vacuum system after each adjustment scan and each imaging scan, with optional exception of the last imaging scan of the magnetic resonance measurement. The predetermined time interval may correspond to a duration of an adjustment scan and/or an imaging scan. It is conceivable that the predetermined time interval varies in dependence of a progression of the magnetic resonance measurement, particularly the duration of an upcoming adjustment scan and/or imaging scan. The predetermined time interval may also correspond to a combined duration of multiple adjustment scans, a combined duration of an adjustment scan and an imaging scan and/or a combined duration of multiple imaging scans.

The processing unit may be configured to determine optimized times at which the imaging sequence can be paused without directly interrupting an acquisition of magnetic resonance signals, while still ensuring compliance with the predetermined time interval.

In pausing the magnetic resonance measurement or the acquisition of magnetic resonance signals after a predetermined time interval and activating the vacuum system, an amount of saliva within the oral cavity of a patient may favourably be maintained below a limit value which triggers or overly intensifies a patient's urge to swallow.

According to an embodiment of the magnetic resonance device, the control unit is configured to activate the vacuum system for a predetermined activation period before or during the imaging sequence and suspend the imaging sequence for the predetermined activation period.

The predetermined activation period may relate to an amount of time required to remove a predetermined amount of saliva from the oral cavity of the patient. The predetermined amount of saliva may be defined by a typical amount of saliva or a fraction of more than 50%, 60%, 70%, 80%, or 90% of a typical amount of saliva inducing an urge to swallow. The predetermined activation period may also depend on a property of the vacuum system and/or the saliva suction device, e. g. a capacity or power of the conveyor unit, a diameter of the fluid pipe connecting the saliva suction device to the vacuum system, a number of suction openings, a mean diameter of the suction openings, and the like.

According to an embodiment, the control unit is configured to activate the vacuum system for the predetermined activation period before or during an imaging sequence. Particularly, the control unit may be configured to suspend an acquisition of magnetic resonance signals for the predetermined activation period.

In suspending an imaging sequence for a predetermined activation period, a build-up of a critical or a limit amount of saliva triggering or inducing an urge to swallow may favourably be avoided.

According to an embodiment, the inventive magnetic resonance device comprises an output device. The output device is configured to output an information about a period of time during which the vacuum system is active or inactive to the patient.

The output device may be configured to output an acoustic, a visual and/or a haptic signal. For example, the output device may be configured to output a noise, a melody, an instruction, a picture, a video and/or a vibration. Preferably, the output provided by the output device is configured to inform the patient about the period of time during which the vacuum system is active or inactive. The output may be self-explanatory or understandable in conjunction with further information, such as an oral instruction by the medical personnel, or a written instruction given to the patient before the magnetic resonance measurement.

Preferably, the information about the period of time during which the vacuum system is active or inactive notifies the patient when it is possible to swallow without compromising the acquisition of magnetic resonance signals. For example, the control unit may be configured to activate or deactivate the vacuum system in dependence of a predetermined schedule of an imaging sequence, particularly a start, an end and/or a duration of an acquisition of magnetic resonance signals and/or a sequence of electromagnetic pulses (e. g. gradient pulses and/or radio-frequency pulses).

It is also conceivable that the information notifies the patient that the vacuum system is active and that it is safe to swallow. Thus, the patient may intentionally swallow to further reduce an amount of saliva in the oral cavity and/or moderate an urge to swallow during a subsequent acquisition of magnetic resonance signals. Furthermore, the output of the output device may be configured to prevent the patient from swallowing during an data acquisition, e. g. by providing an indication when magnetic resonance signals are acquired.

According to an embodiment, the control unit is configured to control the output device to output the information about the period of time during which the vacuum system is active or inactive. The control unit may be connected to the output device via a suitable signal connection. The output device may form a part of the magnetic resonance device, particularly the vacuum system.

An output device configured to output an information about a period of time during which the vacuum system is active or inactive to a patient may favourably make the patient aware of periods during which it is safe to swallow without compromising the magnetic resonance measurement. Furthermore, said output may also help to notify the patient to avoid movements, particularly swallowing motions, during an acquisition of magnetic resonance signals or an upcoming acquisition of magnetic resonance signals.

The invention further relates to a computer-implemented method for removing saliva from an oral cavity of a patient during a magnetic resonance measurement. The method comprises the step of activating and deactivating a vacuum system fluidly connected to a suction opening of a saliva suction device arranged within the oral cavity of the patient in dependence of an imaging sequence of the magnetic resonance measurement.

According to certain embodiments, the inventive method may comprise:
- automatically deactivating the vacuum system for a duration of an acquisition of magnetic resonance signal,
- activating or deactivating the vacuum system in dependence of a trigger signal provided with the imaging sequence, wherein the trigger signal defines one or more time periods during the imaging sequence in which the vacuum system is active or inactive,
- pausing an acquisition of magnetic resonance signals after a predetermined time interval has passed, particularly a time interval of less than 30 s, less than 60 s, less than 90 s, less than 120 s, less than 150 s, less than 180 s, less than 210 s, less than 240 s, less than 270 s, or less than 300 s, and activate the vacuum system for a predetermined period of time before resuming the acquisition of magnetic resonance signals,
- activating the vacuum system for a predetermined activation period before or during the imaging sequence and suspend the imaging sequence for the predetermined activation period, and/or
- outputting an information about a period of time during which the vacuum system is active or inactive to the patient.

The saliva suction device may be embodied in accordance with an embodiment described herein. Preferably, the inventive magnetic resonance device according to an embodiment described above is configured to carry out one or more embodiments of the inventive method.

The inventive method shares the advantages of the inventive magnetic resonance device according to an embodiment described above.

The invention further relates to a computer program product that can be loaded into a memory of a programmable processing unit of a magnetic resonance device according to an embodiment described above. The inventive computer program product comprises program code means to perform an inventive method according to an embodiment described above when the computer program product is executed in the processing unit of the magnetic resonance device.

As a result, the method according to the invention can be carried out quickly and in a robust and repeatable manner. The computer program product is configured in such a way that it can carry out the inventive method by means of the processing unit. The processing unit comprises prerequisites, such as a main memory, a graphics card, or a logic unit, required to carry out certain embodiments of the inventive method.

The computer program product is, for example, stored on a computer-readable medium or stored on a network, a server, or a cloud, from where it can be loaded into the processor of a local processing unit. The local processing unit can be directly connected to the magnetic resonance device or designed as part of the magnetic resonance device, e. g. a part of a control unit of the magnetic resonance device. Furthermore, control information of the computer program product can be stored on an electronically readable medium. The control information on the electronically readable medium can be designed in such a way that, when the medium is used, it carries out a method according to the invention in a processing unit of the magnetic resonance device. Examples of an electronically readable medium are a DVD, a magnetic tape, or a USB stick on which electronically readable control information, in particular software, is stored. If this control information is read from the medium and stored in a control unit and/or a processing unit of a magnetic resonance device, all embodiments of the inventive method described above can be carried out.

Further advantages and details of the present invention may be recognized from the embodiments described below as well as the drawings. The figures show:
- Fig. 1: a schematic representation of an embodiment of an inventive magnetic resonance de-vice,
- Fig. 2: a schematic representation of an embodiment of an inventive saliva suction device,
- Fig. 3: a schematic representation of an embodiment of an inventive combined intraoral suction device,
- Fig. 4: a schematic representation of an embodiment of an inventive combined intraoral suction device,
- Fig. 5: a schematic representation of an embodiment of an inventive combined intraoral suction device,
- Fig. 6: a schematic representation of an embodiment of an inventive combined intraoral suction device,
- Fig. 7: a schematic representation of an embodiment of an inventive combined intraoral suction device,
- Fig. 8: a schematic representation of an embodiment of an inventive combined intraoral suction device,
- Fig. 9: a schematic representation of an embodiment of an inventive magnetic resonance de-vice.

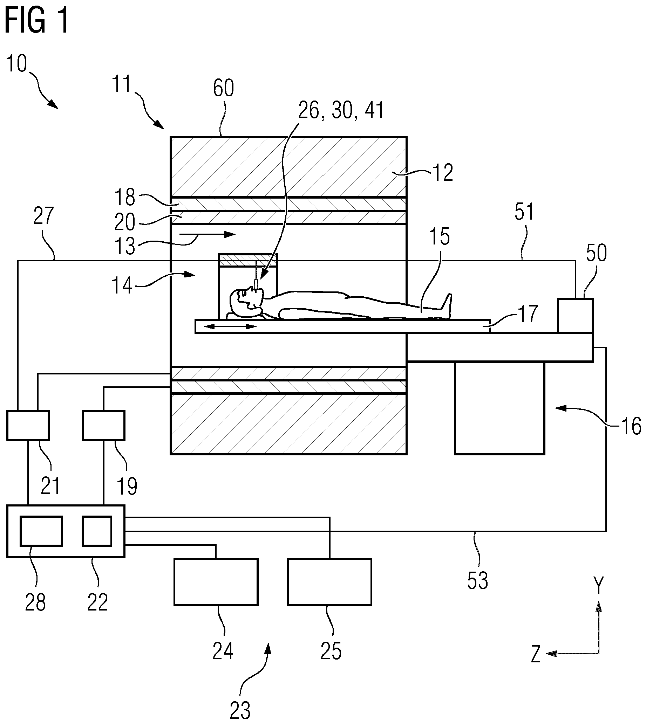

Fig. 1 shows an embodiment of a magnetic resonance device 10 according to the invention. In the illustrated example, the magnetic resonance device 10 comprises a static field magnet or main magnet 12 configured to provide a homogenous, static magnetic field 13 (B0 field). The static magnetic field 13 permeates an imaging region 14 configured to receive an imaging object, such as a patient 15. The imaging region 14 may correspond to a patient bore configured to accommodate the patient 15 during a magnetic resonance measurement. The imaging region 14 is encompassed by the main magnet 12 in a circumferential direction.

The magnetic resonance device 10 may comprise a patient positioning device 16 configured to transport the patient 15 into the imaging region 14. Particularly, the patient positioning device 16 may be configured to transport a diagnostically relevant body region of the patient 15 into an imaging volume or isocentre (not shown) of the magnetic resonance device 10. The main magnet 12 and other components of the magnetic resonance device 10 may be concealed in a housing 60.

The magnetic resonance device 10 may comprise a gradient system including one or more gradient coils 18. The one or more gradient coils may be configured to generate gradient magnetic fields in different, preferably orthogonally oriented, spatial directions. The gradient magnetic fields can be used for spatial encoding of magnetic resonance signals or magnetic resonance data acquired during a magnetic resonance measurement. The one or more gradient coils 18 can be activated or controlled via a control signal provided by a gradient control unit 19.

The magnetic resonance device 10 may further comprise an integrated radio-frequency antenna 20 (i. e. a body coil). The radio-frequency antenna 20 may be activated or controlled via a radio-frequency control unit 21. The radio-frequency control unit 21 may be configured to control the radio-frequency antenna 20 to generate a high frequency magnetic field (B1-field) and emit radio-frequency excitation pulses into the imaging region 14. The magnetic resonance device 10 may also comprise a local coil 26. The local coil 26 may be positioned in proximity to a diagnostically relevant region, particularly a jaw region or dental region, of the patient 15. The local coil 26 may be configured to emit radio-frequency excitation pulses into the patient 15 and/or receive magnetic resonance signals from the patient 15. It is conceivable, that the body coil 20 and the local coil 26 are controlled via the radio-frequency controller 21.

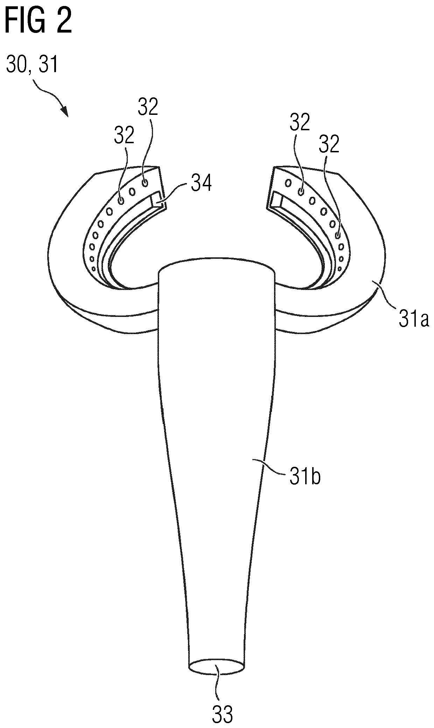

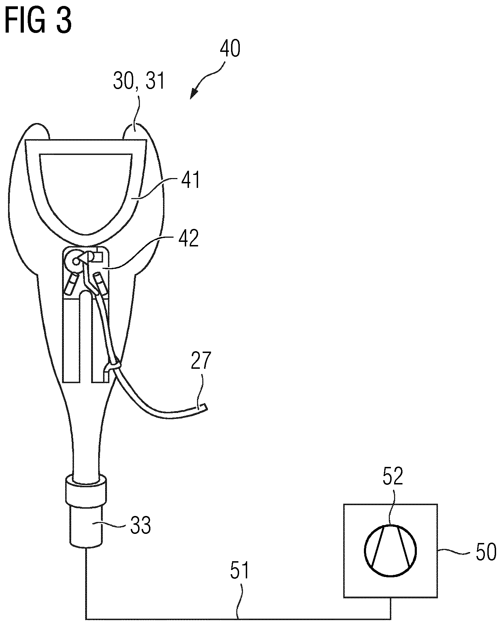

In the depicted example, the local coil 26 comprises an intraoral radio-frequency coil 41 (see Figs. 3 to 8) configured to be inserted into the oral cavity of the patient 15. The intraoral radio-frequency coil 41 is connected to the radio-frequency controller 21 via the electrical connection cable 27. The local coil 26 further comprises a saliva suction device 30 (see Figs. 2 to 8) configured to remove saliva from the oral cavity of the patient 15. The saliva suction device 30 may be connected to a vacuum system 50 via a fluid pipe 51. The vacuum system 50 may be portable. However, the vacuum system 50 may also be mechanically and/or electrically integrated within the magnetic resonance device 10, particularly the patient positioning device 16. The intraoral radio-frequency coil 41 may be reversibly or irreversibly attached to the saliva suction device 30. Preferably, the saliva suction device 30 and the intraoral radio-frequency coil 41 form a combined intraoral suction device 40 in accordance with an embodiment depicted in the Figs. 3 to 8.

The magnetic resonance device 10 comprises a control unit 22 configured to control the magnetic resonance device 10. The control unit 22 may comprise a processing unit 28 configured to process magnetic resonance signals and reconstruct magnetic resonance images. The processing unit 28 may also be configured to process input from a user of the magnetic resonance device 10 and/or provide an output to a user. For this purpose, the processing unit 28 and/or the control unit 22 can be connected to a display unit 24 and an input unit 25 via a suitable signal connection. For a preparation of a magnetic resonance measurement, preparatory information, such as imaging parameters or patient information, can be provided to the user via the display unit 24. The input unit 25 may be configured to receive information and/or imaging parameters from the user.

In the embodiment depicted in Fig. 1, the control unit 22 is configured to activate or deactivate the vacuum system 50 by transmitting a control signal via the electrical signal line 53. When the vacuum system 50 is deactivated or switched-off, a pressure at a suction opening of the saliva suction device 30 may correspond to an atmospheric pressure or a pressure within the oral cavity of the patient 15. However, the pressure at the suction opening of the saliva suction device 30 may drop to a negative pressure when the vacuum system 50 is active. Preferably, the control unit 22 is configured to activate and deactivate the vacuum system 50 in dependence of an imaging sequence of a magnetic resonance measurement.

The inventive magnetic resonance device 10 is configured to perform a magnetic resonance measurement of the patient 10, particularly a magnetic resonance measurement of a dental region of the patient 15, positioned within the imaging region 14. In a preferred embodiment, the local coil 26 is configured as an intraoral radiofrequency coil 41 attached to a saliva suction device 30 having a body 31 comprising one or more suction openings 32 (see Figs. 3 to 8) fluidly connected to the vacuum system 50 via the fluid pipe 51. The control unit 22 is configured to activate and deactivate the vacuum system 50 in dependence of an imaging sequence of the magnetic resonance measurement. Particularly, the control unit 22 may be configured to automatically deactivate the vacuum system 50 for a duration of an acquisition of magnetic resonance signals.

For example, the control unit 22 may be configured to deactivate or switch-off the vacuum system 50 via a control signal transmitted via the electrical signal line 53 one or more times over the course of an imaging sequence. Thus, the imaging sequence may proceed as usual, but the vacuum system 50 may be deactivated one or more times to avoid movement of tissue or fluid in the oral cavity while magnetic resonance signals are acquired.

In a further example, the control unit 22 may be configured to activate or deactivate the vacuum system 50 in dependence of a trigger signal provided with an imaging sequence. The trigger signal may be determined and/or provided by the processing unit 28 and/or the control unit 22. However, the trigger signal may also correspond to a specific electromagnetic pulse transmitted via the gradient control unit 19 and/or the radio-frequency control unit 21. The control unit 22 and/or processing unit 28 may be configured to activate or deactivate the vacuum system 50 in dependence of such specific electromagnetic pulse.

The trigger signal may depend on the imaging sequence to be carried out. For example, different trigger signals may be provided with different imaging sequences. Preferably, the trigger signal defines or denotes one or more time periods during the imaging sequence in which the vacuum system 50 should be active or switched-off.

According to a further embodiment, the control unit 22 is configured to pause an acquisition of magnetic resonance signals after a predetermined time interval has passed, particularly a time interval of less than 30 s, less than 60 s, less than 90 s, or less than 120 s, and activate the vacuum system 50 for a predetermined period of time before resuming the acquisition of magnetic resonance signals.

For example, the control unit 22 is configured to change or alter a predetermined schedule of an imaging sequence by pausing or interrupting the imaging sequence and activating the vacuum system 50 for the predetermined period of time. After the predetermined period of time has expired, the control unit 22 may deactivate or switch-off the vacuum system 50 and resume the interrupted or paused imaging sequence. Thus, a critical build-up of saliva in the oral cavity of the patient 15 may be avoided and magnetic resonance signals may be acquired without the patient 15 having to suppress an urge to swallow for an elongated period of time.

In still a further embodiment, the control unit 22 is configured to activate the vacuum system 50 for a predetermined activation period before or during an imaging sequence and suspend the imaging sequence for the predetermined activation period.

Of course, the magnetic resonance device 10 may comprise further components and/or functions which are common in magnetic resonance devices. The general operation of a magnetic resonance device 10 is known to those skilled in the art, so a more detailed description is omitted.

Fig. 2 depicts an embodiment of an inventive saliva suction device 30. The saliva suction device 30 comprises a body 31 configured to be inserted into the oral cavity of a patient 15. It is conceivable that just a section 31a of the saliva suction device 30 is configured to be inserted into the oral cavity. The body 31 comprises a plurality of suction openings 32 connected to the fluid pipe 51 via the port 33. Preferably, the saliva suction device 30 is shaped in such a way that the suction openings 32 are arranged between teeth and an inner check and/or between teeth and a tongue of the patient 15 when the saliva suction device is properly arranged within the oral cavity of the patient 15.

A section 31a of the body 31 is shaped in such a way to encompass at least a part of an intraoral radio-frequency coil 41 (see Figs. 3 to 8) and to provide a form-locking and/or force locking mechanical connection with the intraoral radio-frequency coil 41. In the depicted example, the plier-shaped part 31a is configured to accommodate and encompass an intraoral radio-frequency coil 41 from two opposing sides and hold the intraoral radio-frequency coil 41 firmly in place.

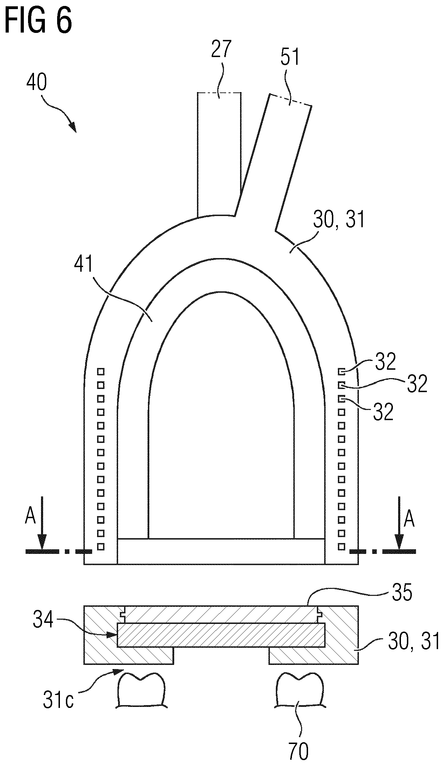

The body 31 of the saliva suction device 30 may also comprise a receiving section 34 configured to receive a section, particularly an edge, of an intraoral coil 41 (see Fig. 6). The receiving section 34 may be configured to provide a form-locking mechanical connection between the saliva suction device 30 and the intraoral radio-frequency coil 41 and prevent any movement of the intraoral radio-frequency coil 41 relative to the saliva suction device 30 in a direction orthogonal to a plane defined by the part 31a of the body 31.

Fig. 3 shows an embodiment of an inventive combined intraoral suction device 40. The combined intraoral suction device 40 comprises a saliva suction device 30 reversibly or irreversibly attached to an intraoral radio-frequency coil 41. The section of the combined intraoral suction device 40 comprising the intraoral radio-frequency coil 41 may be shaped in such a way to be arranged within the oral cavity of a patient 15. Preferably, the intraoral radio-frequency coil 41 comprises a substantially planar or flat shape configured to be arranged in an occlusal plane between the two dental arches of the patient 15.

The intraoral radio-frequency coil 41 may comprise an electronic circuit 42 in accordance with an embodiment described above. The electronic circuit 42 may protrude from the oral cavity of the patient 15 or be arranged outside of the oral cavity of the patient 15 when the intraoral radio-frequency coil is properly arranged within the oral cavity of the patient 15 for a magnetic resonance measurement. In the depicted example, the electronic circuit 42 is attached to the body 31 of the saliva suction device 30, however, the electronic circuit 42 may also be at least partially embedded within the body 31 of the saliva suction device 30. The electronic circuit 42 electrically connects the intraoral radio-frequency coil 41 to the radio-frequency control unit 21 (see Fig. 1) via the electrical connection cable 27. The electronic circuit 42 may also comprise a wireless communication unit configured to wirelessly send magnetic resonance data to the control unit 22 and/or the processing unit 28 of a magnetic resonance device 10. In an alternative embodiment, the intraoral radio-frequency coil 41 is inductively coupled to an external radio-frequency coil, particularly an extraoral radio-frequency coil encompassing a section of a jaw region of the patient 15.

The port 33 fluidly connects the one or more suction openings 32 (not shown) of the saliva suction device 30 to the vacuum system 50 via the fluid pipe 51. The vacuum system 50 comprises a conveyor unit 52, preferably a compressor, configured to evacuate the fluid pipe 51 and provide a negative pressure at the one or more suction openings 32. The saliva removed from the oral cavity of the patient 15 may be separated from the fluid pipe 51 via a suitable siphon arrangement (not shown).

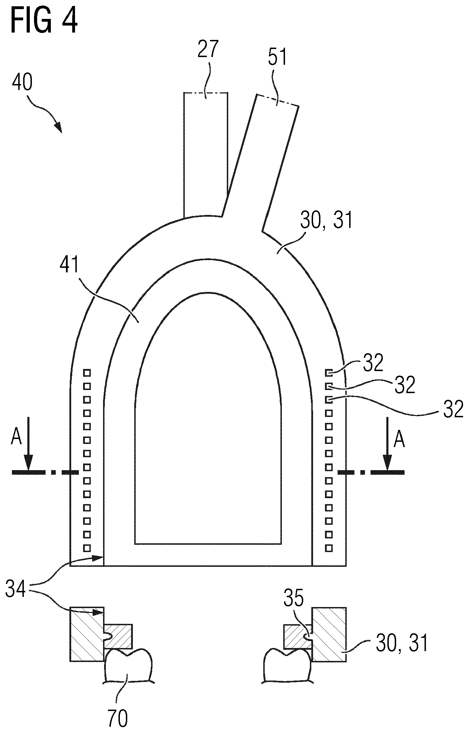

Fig. 4 shows an embodiment of an inventive combined intraoral suction device 40 comprising a saliva suction device 30 reversibly attached to an intraoral radio-frequency coil 41. In the depicted example, the body 31 of the saliva suction device 30 comprises a U-shaped section configured to encompass an outer edge or rim of the intraoral radio-frequency coil 41. The U-shaped section may correspond to a receiving section 34 of the saliva suction device 30.

In addition, the body 31 of the saliva suction device 30 comprises a retaining element 35. The retaining element 35 may correspond to a plurality of nipples or a ridge forming a continuous or discontinuous line along an inner surface of the U-shaped receiving section 34 of the saliva suction device 30. In the depicted example, the intraoral radio-frequency coil 41 comprises a groove shaped complementary to the retaining element 35 of the saliva suction device 30. The groove allows for a mechanical engagement between the intraoral radio-frequency coil 41 and the retaining element 35. Particularly, the groove allows for sliding the intraoral radio-frequency coil 41 into the receiving section 34 of the saliva suction device 30 along a direction oriented orthogonal to a plane defined by the cross-sectional area A-A. The retaining element 35 provides a form-locking and/or force locking mechanical connection between the saliva suction device 30 and the intraoral radio-frequency coil 41 and prevents a relative movement between the saliva suction device 30 and the intraoral radio-frequency coil 41 in any direction oriented in parallel to the plane defined by the cross-sectional area A-A.

Parts of the body 31 of the saliva suction device 30 may be configured to encompass one or more sections of a dental arch of the patient 15. Thus, the body 31 may act as a spacer between an inner surface of a cheek and the teeth 70 of the patient 15, which may prevent the suction openings 32 from catching on to soft tissues within the oral cavity and allow for a more efficient removal of saliva. Furthermore, a process of arranging the intraoral radio-frequency coil 41 in a proper position for a magnetic resonance measurement may favourably be facilitated, as the body 31 may be pushed over a dental arch of the patient 15 to provide a proper relative position between the dental arch and the intraoral radio-frequency coil 41. Of course, the body 31 and the intraoral radio-frequency coil 41 of the combined intraoral suction device 40 may be provided in different sizes to accommodate for differences in sizes of the dentition in children and adults of all ages.

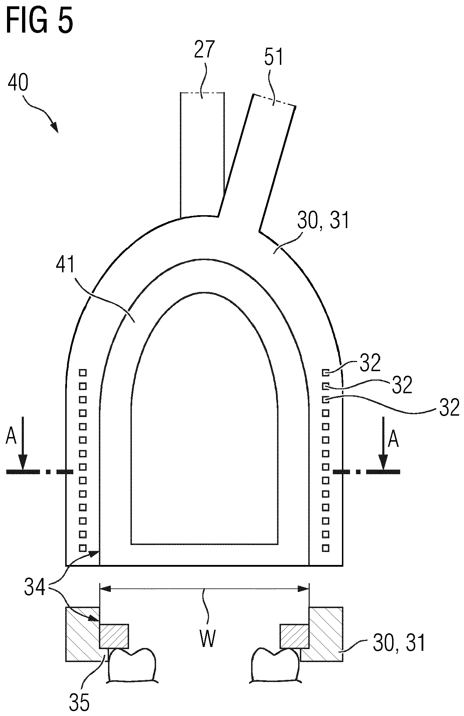

Fig. 5 shows a further embodiment of an inventive combined intraoral suction device 40 comprising a saliva suction device 30 reversibly attached to an intraoral radio-frequency coil 41. In the depicted example, a width W of the receiving section 34 is slightly undersized with respect to a width of the intraoral radio-frequency coil 41, thus providing a force-locking mechanical connection between the body 31 of the saliva suction device 30 and the intraoral radio-frequency coil 41 when the intraoral radio-frequency coil 41 is pushed into the receiving section 34. Preferably, the body 31 of the saliva suction device 30 consists of an elastic material or a flexible material configured to be stretched over a section of the intraoral radio-frequency coil 41, thus holding the intraoral radio-frequency coil 41 in place via elastic restoring forces of the elastic material.

As depicted in Fig. 5, the body 31 of the saliva suction device 30 may comprise a retaining section 35 configured to prevent the intraoral radio-frequency coil 41 from being pushed through an opening provided by the receiving section 34. Thus, the retaining element 35 may support or facilitate a process of attaching the intraoral radio-frequency coil 41 to the saliva suction device 30.

Fig. 6 shows a variant of the combined intraoral suction device 40 illustrated in Fig. 5. In the depicted example, the retaining element 35 of the saliva suction device 30 is configured to mechanically engage with a section of the body 31 of the saliva suction device 30 and to cover the U-shaped receiving section 34 at least partially to prevent the intraoral radio-frequency coil 41 from detaching from the body 31. For example, the retaining element 35 may be configured to slide into a groove provided along an inner surface of the U-shaped receiving section 34 along a direction oriented orthogonally to a plane defined by the cross-sectional area A-A.

It is also conceivable that the saliva suction device 30 comprises a hinge or joint (not shown) allowing the retaining element 35 to be folded over an opening provided by the receiving section 34. For example, the hinge or joint may be configured to fold or close the retaining element 35 over a lateral surface (i. e. a surface oriented orthogonally to the plane defined by the cross-sectional area A-A) or a transversal surface (i. e. a surface oriented in parallel to the plane defined by the cross-sectional area A-A) of the saliva suction device 30. The retaining element 35 may further comprise a securing element (not shown), such as a pin, a bolt, a strap and/or a flap, configured to hold the retaining element 35 in place.

In the embodiment shown in Fig. 6, a section 31c of the body 31 of the saliva suction device 30 is arranged between the teeth 70 and the intraoral radio-frequency coil 41. The section 31c is configured to encompass at least a part of the intraoral radio-frequency coil 41 and to provide a protective cover for the intraoral radio-frequency coil 41. For example, the section 31c is configured to prevent the teeth 70 from making direct mechanical contact with the intraoral radio-frequency coil 41.

According to an embodiment, the section 31c comprises or consists of an electrical insulator configured to prevent an electrical current to be transmitted from the intraoral radio-frequency coil 41 to the teeth 70 or other tissues in the oral cavity of the patient 15. However, it is also conceivable that the intraoral radio-frequency coil 41 comprises a protective cover or coating configured to prevent a direct mechanical contact and/or a transmission of electrical currents between the intraoral radio-frequency coil 41 and the teeth 70.

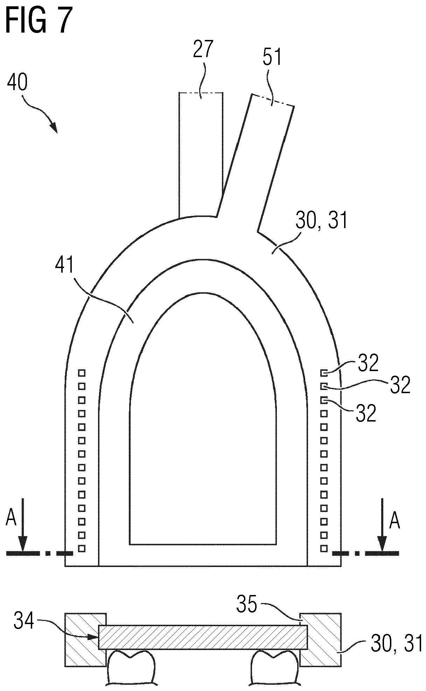

Fig. 7 illustrates a further embodiment of an inventive combined intraoral suction device 40 comprising a saliva suction device 30 reversibly attached to an intraoral radio-frequency coil 41. In the depicted example, the receiving section 34 is formed as a groove along an inner surface of the U-shaped section of the body 31 of the saliva suction device 30. Ridges bordering or surrounding the groove or receiving section 34 may act as retaining elements 35 holding the intraoral radio-frequency coil 41 in place and preventing the intraoral radio-frequency coil 41 from being moved relative to the body 31 in a direction oriented in parallel to a plane defined by the cross-sectional area A-A.

To attach the intraoral radio-frequency coil 41 to the saliva suction device 30, the intraoral radio-frequency coil 41 may be slid into the receiving section 34 of the saliva suction device 30 along a direction oriented orthogonal to the plane defined by the cross-sectional area A-A.

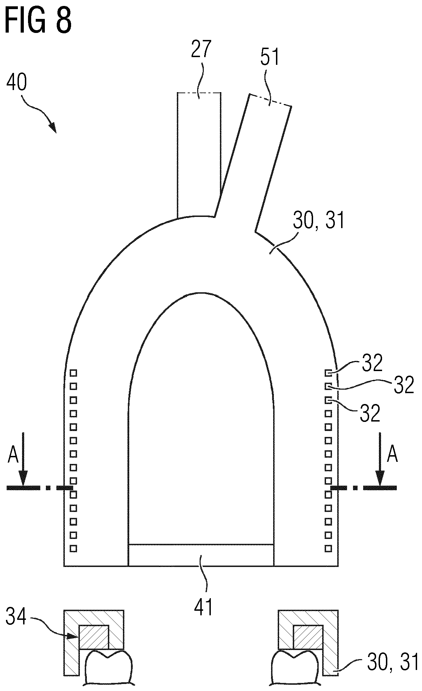

Fig. 8 shows further embodiment of an inventive combined intraoral suction device 40. In the present example, the receiving section 34 is configured as a U-shaped groove in a lateral surface of the body 31 of the saliva suction device 30. The combined intraoral suction device 40 may comprise a retaining element 35 (not shown) configured to hold the intraoral radio-frequency coil 41 in a predefined position relative to the body of the saliva suction device 30. For example, the retaining element 35 may be configured to cover the groove provided by the receiving section 34 and prevent the intraoral radio-frequency coil 41 from moving in a direction oriented in parallel to the plane defined by the cross-sectional area A-A. However, it is also conceivable that a dimension of the receiving section 34 is slightly undersized in comparison to a dimension of the intraoral radio-frequency coil 41 and provides a force-locking mechanical connection with the intraoral radio-frequency coil 41, when the intraoral radio-frequency coil 41 is pushed into the receiving section 34. Additionally or alternatively, a surface of the receiving section 34 may comprise a rough or sticky coating configured to engage with a surface of the intraoral radio-frequency coil 41 and prevent a detaching of the intraoral radio-frequency coil 41 from the body 31 of the saliva suction device 30.

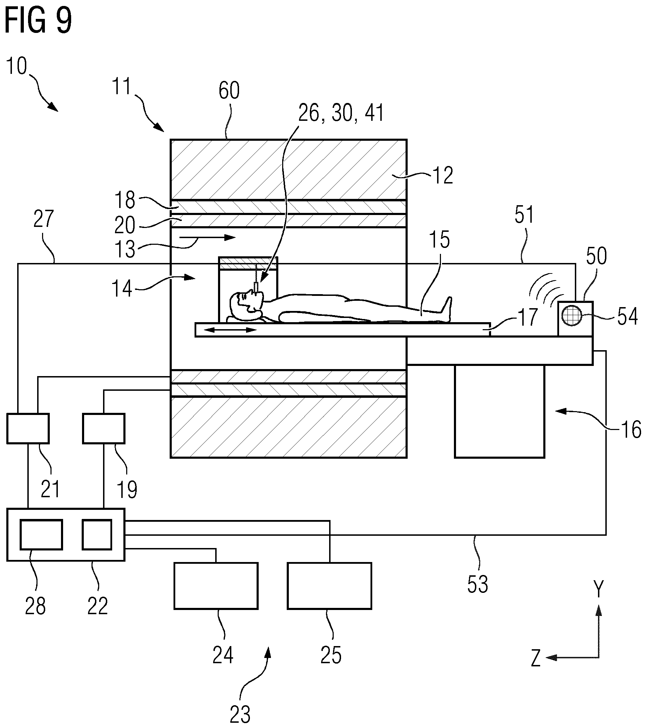

Fig. 9 shows an embodiment of the inventive magnetic resonance device 10 comprising an output device 54 configured to output an information about a period of time during which the vacuum system 50 is active or inactive to the patient 15. In the depicted example, the output device 54 comprises a speaker configured to provide a noise or an instruction informing the patient 15 whether the vacuum system 50 is active or not. For example, the output device 54 may provide an alarm signal signifying that the vacuum system 50 is switched-off and that the patient 15 is not allowed to swallow. In a further example, the output device 54 may output a voice message notifying the patient 15 that the vacuum system 50 is active and that the patient 15 is allowed to swallow.

According to an embodiment, the control unit 22 is configured to control the output device 54 to output the information about a period of time during which the vacuum system 50 is active or inactive to the patient 15. However, the output device 54 may also be configured to output the information about a period of time during which the vacuum system 50 is active or inactive in dependence of an operating status of the vacuum system 50 and/or a signal received via the electrical connection line 53.

The embodiments described herein are to be recognized as examples. It is to be understood that individual embodiments may be extended by or combined with features of other embodiments if not explicitly stated otherwise. The embodiments depicted in the Figs. 1 to 9 are representations that do not necessarily have to be to scale.

## Claims

1. A saliva suction device (30) for an intraoral radio-frequency coil (41), comprising a body (31) and a suction opening (32) configured to be connected to a vacuum system (50), wherein the body (31) is configured to provide a mechanical connection between the saliva suction device (30) and the intraoral radio-frequency coil (41), wherein the intraoral radio-frequency coil (41) is configured to receive magnetic resonance signals from a dental region of a patient (15), and wherein the suction opening (32) is configured to remove saliva from an oral cavity of the patient (15) during a magnetic resonance measurement.

2. The saliva suction device (30) according to claim 1, wherein the body (31) is configured to encompass at least a part of the intraoral radio-frequency coil (41) and/or provide a protective cover for at least a part of the intraoral radio-frequency coil (41).

3. The saliva suction device (30) according to one of the claims 1 or 2, wherein the body (31) is shaped in such a way to encompass at least a part of the intraoral radio-frequency coil (41) and to provide a form-locking and/or force locking mechanical connection with the part of the intraoral radio-frequency coil (41).

4. The saliva suction device (30) according to one of the preceding claims, wherein the body (31) comprises a receiving section (34) shaped complementary to a section of the intraoral radio-frequency coil (41) in such a way to provide a form-locking mechanical connection between the section of the intraoral radio-frequency coil (41) and the receiving section (34) of the saliva suction device (30).

5. The saliva suction device (30) according to one of the preceding claims, comprising a retaining element (35) configured to hold the intraoral radio-frequency coil (41) in a predefined relative position to the saliva suction device (30).

6. The saliva suction device (30) according to one of the preceding claims, wherein the body (31) comprises or consists of an elastic material configured to allow the body (31) to reversibly deform under manual effort, and wherein the body (31) is configured to attach to and/or detach from the intraoral radio-frequency coil (41) under reversible deformation.

7. The saliva suction device (30) according to one of the preceding claims, further comprising a portable vacuum system (50) fluidly connected to the suction opening.

8. A combined intraoral suction device (40), comprising a saliva suction device (30) according to one of the preceding claims, and an intraoral radio-frequency coil (41) attachable to the saliva suction device (30) and configured to receive magnetic resonance signals from a dental region of a patient (15) when the combined intraoral suction device (40) is properly arranged within an oral cavity of the patient (15) for a magnetic resonance measurement of the dental region.

9. A magnetic resonance device (10) configured to perform a magnetic resonance measurement of a dental region of a patient (15) positioned within an imaging region of the magnetic resonance device (10), comprising a control unit (22), a saliva suction device (30), and a vacuum system (50) connected to a suction opening (32) of the saliva suction device (30), wherein the suction opening (32) is configured to remove saliva from an oral cavity of the patient (15), and wherein the control unit (22) is configured to activate and deactivate the vacuum system (50) .

10. The magnetic resonance device (10) according to claim 9, wherein the saliva suction device (30) forms a part of a combined intraoral suction device (40) according to claim 8.

11. The magnetic resonance device (10) according to one of the claims 9 or 10, wherein the control unit (22) is configured to activate and deactivate the vacuum system (50) in dependence of an imaging sequence of the magnetic resonance measurement.

12. The magnetic resonance device (10) according to claim 11, wherein the control unit (22) is configured to automatically deactivate the vacuum system (50) for a duration of an acquisition of magnetic resonance signals.

13. The magnetic resonance device (10) according to one of the claims 11 or 12, wherein the control unit (22) is configured to activate or deactivate the vacuum system (50) in dependence of a trigger signal provided with the imaging sequence, wherein the trigger signal defines one or more time periods during the imaging sequence in which the vacuum system (50) is active or inactive.

14. The magnetic resonance device (10) according to one of the claims 11 to 13, wherein the control unit (22) is configured to pause an acquisition of magnetic resonance signals after a predetermined time interval has passed, particularly a time interval of less than 30 s, less than 60 s, less than 90 s, less than 120 s, less than 150 s, less than 180 s, less than 210 s, less than 240 s, less than 270 s, or less than 300 s, and activate the vacuum system (50) for a predetermined period of time before resuming the acquisition of magnetic resonance signals.

15. The magnetic resonance device (10) according to one of the claims 11 to 14, wherein the control unit (22) is configured to activate the vacuum system (50) for a predetermined activation period before or during the imaging sequence and suspend the imaging sequence for the predetermined activation period.

16. The magnetic resonance device (10) according to one of the claims 11 to 15, further comprising an output device (54) configured to output an information about a period of time during which the vacuum system (50) is active or inactive to the patient (15).
